# FASCICULE DE BREVET EUROPEEN

(11) **EP 1 284 688 B1**
(45) Date de publication et mention de la délivrance du brevet: **26.10.2005**
(21) Numéro de dépôt: 00976200.6
(22) Date de dépôt: 07.11.2000
(51) Int. Cl.: A61F 2/24, A61L 27/58

(54) **DISPOSITIF POUR RETRECIR ET/OU RENFORCER LES ORIFICES VALVULAIRES DU COEUR**
VORRICHTUNG ZUR REDUZIERUNG UND/ODER VERSTÄRKUNG DER HERZKLAPPENÖFFNUNGEN
DEVICE FOR SHRINKING OR REINFORCING THE HEART VALVULAR ORIFICES

(30) Priorité: 25.05.2000 WO PCT/IB00/00707
(43) Date de publication de la demande: 26.02.2003
(73) Titulaire: Bioring SA, 1027 Lonay (CH); Kalangos, Afksendiyos, CH-1208 Geneve (CH)
(72) Inventeur: KALANGOS, Afksendiyos, CH-1208 Geneve (CH); ANDRIEU, Raymond, CH-1137 Yens (CH); LE GOFF, Philippe, CH-1052 Le Mont sur Lausanne (CH)
(74) Mandataire: Micheli & Cie
(86) Numéro de dépôt international: PCT/IB2000/001605
(87) Numéro de publication internationale: WO 2001/089426

(56) Documents cités:
- EP-A- 0 338 994
- EP-A- 0 594 148
- WO-A-96/04852
- WO-A-97/16135
- US-A- 4 917 698
- US-A- 4 923 470

## Description

Les lésions des orifices valvulaires du coeur que ce soit des valvules sigmoïdes de l'aorte ou de l'artère pulmonaire ou que ce soit des valves mitrales ou tricuspides proviennent dans 80 à 90% des cas d'un prolapsus ou d'une restriction qui induisent une dilatation de l'anneau en élargissant les cavités cardiaques concernées et dans pratiquement tout le reste des cas d'une dilatation de l'anneau sans lésion valvulaire associée.

Après la correction des lésions valvulaires associées, il est nécessaire de corriger en même temps la dilatation de l'anneau valvulaire et de le maintenir à sa dimension normale. Pour prévenir la récidive de telles lésions, il est nécessaire de renforcer l'anneau entourant ces orifices valvulaires.

Pour réaliser de telles réparations des orifices valvulaires, on a proposé plusieurs types d'implants rigides ou flexibles présentant la forme générale d'anneaux, tels les anneaux DURAN, CARPENTIER ou PUIG-MASSANA ou des segments, tel celui de COSGROVE. Ces anneaux ou segments rigides ou flexibles sont disposés et cousus le long du pourtour de l'anneau de l'orifice valvulaire à réparer. L'ouverture de l'orifice valvulaire est ainsi ramenée à la dimension voulue, généralement calculée en proportion de la surface du corps du patient, et maintenue à cette dimension normale.

Le document EP 0 338 994 expose un tel dispositif pour la correction chirurgicale de l'insuffisance tricuspide destiné à être fixé, spécifiquement cousu, le long du pourtour de l'anneau de l'orifice valvulaire à réparer, ceci de manière habituelle sur la surface intérieure de cet orifice valvulaire. Afin d'accélérer le processus de fixation par suture, ce dispositif peut être équipé à ses extrémités d'un fil et d'une aiguille. Ce fil et l'aiguille correspondants ne servent cependant pas à d'autres fins que la suture plus rapide, une fois l'insertion du dispositif dans le coeur ayant été complétée, et le dispositif reste donc dans le contexte d'implants et de techniques d'implantations connus en n'améliorant que la vitesse d'un geste chirurgical conventionnel.

Ces anneaux implantés sont généralement en matière synthétique ou en métal et peuvent prédisposer certains patients à des infections valvulaires en cas de bactériémies nécessitant des traitements thérapeutiques curatifs et préventifs, et le cas échéant une nouvelle intervention.

En outre, lorsque ces anneaux rigides ou flexibles sont utilisés chez des enfants ou des nourrissons ils empêchent la croissance normale de l'anneau de l'orifice valvulaire concerné, ce qui conduit à des sténoses et également à une ou plusieurs nouvelles interventions successives pour élargir l'anneau et remplacer la valve sténosée.

Le document WO 97/16135 décrit une prothèse cardiaque annulaire résorbable. L'anneau exposé dans ce document est formé d'un matériau biodégradable afin de permettre le remplacement de ce matériau par du matériau biologique propre au patient lors de la période de résorption. Pourtant, cet anneau est également destiné à être disposé de manière habituelle sur la surface intérieure de l'orifice valvulaire le long du pourtour de l'anneau de cet orifice valvulaire à réparer.

La présente invention a pour objet un dispositif pour rétrécir et/ou renforcer les orifices valvulaires du coeur qui évite toute prédisposition à l'infection et qui permette la croissance normale de l'anneau de la valve chez l'enfant, évitant ainsi les sténoses ou les interventions successives; de plus, le dispositif est agencé de manière à permettre un geste chirurgical tout à fait nouveau et innovant et améliore ainsi grandement la vitesse, la facilité et l'applicabilité de l'intervention chirurgicale.

Le dispositif pour rétrécir et/ou renforcer les orifices valvulaires du coeur se distingue par les caractéristiques énumérées à la revendication 1 et/ou les revendications dépendantes.

Le dessin annexé illustre schématiquement et à titre d'exemple plusieurs formes d'exécution du dispositif selon l'invention.

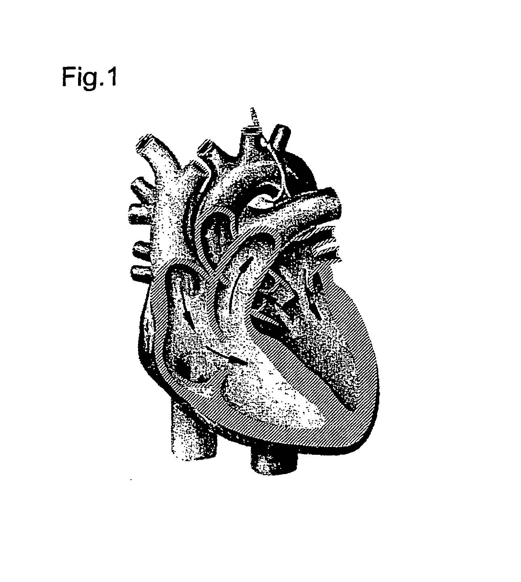

La figure 1 est une représentation schématique du coeur humain. Les figures 2, 3 et 4 sont des schémas simplifiés illustrant respectivement ta valve mitrale, la valve tricuspide et les valvules sigmoïdes.
La figure 5 illustre de façon schématique ou simplifiée une première forme d'exécution du dispositif selon l'invention.
La figure 6 illustre de façon schématique ou simplifiée une seconde forme d'exécution du dispositif selon l'invention.
La figure 7 illustre de façon schématique ou simplifiée une troisième forme d'exécution du dispositif selon l'invention.
La figure 8 illustre de façon schématique ou simplifiée une quatrième forme d'exécution du dispositif selon l'invention.
La figure 9 illustre de façon schématique ou simplifiée une variante du dispositif illustré à la figure 7.

A la place de fixer rigidement et/ou définitivement un anneau ou segment rigide ou flexible le long de tout ou partie de la périphérie de l'orifice valvulaire, comme cela se fait jusqu'ici, la présente technique consiste à disposer un lien souple le long de tout ou partie de la périphérie de l'orifice valvulaire à l'intérieur de l'endocarde, soit la couche de tissus située du côté interne du muscle myocarde, à définir la dimension de l'ouverture valvulaire par une longueur définie du lien, à fixer celui-ci en un ou deux points de l'endocarde par exemple par des points de suture.

De plus, dans la présente technique, on utilise un lien résorbable (biodégradable ou bioabsorbable) c'est à dire biodégradable et n'entraînant pas de réponse immunitaire de la part de l'organisme. Dans la suite du document on utilise le terme résorbable pour définir de façon indifférenciée bioabsorbable ou biodégradable et des fils de suture résorbables ou non. Dans un premier temps après l'opération, le lien maintient l'anneau valvulaire à sa dimension normale ou voulue empêchant ainsi sa dilatation.

Puis, par l'action de résorption du lien à l'intérieur de la couche endomyocardique, l'organisme crée par réaction une cicatrice le long du lien caractérisé par du tissu fibreux présentant une plus grande résistance à l'étirement. Ainsi, une fois le lien résorbé par l'organisme, le maintien de l'orifice valvulaire à la dimension voulue est effectué par la rigidité de ce tissu fibreux de la cicatrice.

Comme la cicatrice résiduelle est constituée de tissus biologiques propres au patient, il ne peut y avoir de prédisposition à l'infection, mais surtout cette cicatrice peut grandir normalement au cours du processus de croissance de l'enfant, ce qui évite les problèmes de sténoses tardives.

Cette nouvelle technique est rendue possible par le dispositif de réparation des lésions, de rétrécissement et/ou de renforcement des orifices valvulaires du coeur selon la présente invention. Dans une première forme d'exécution ce dispositif comporte un lien 1, réalisé dans un matériau résorbable par l'organisme, terminé à l'une de ses extrémités par une boucle 2 ou un organe d'arrêt et de fixation résorbable ou non, par exemple un crochet ou hameçon permettant la fixation de cette extrémité à l'endomyocarde. L'autre extrémité du lien 1 est solidaire, généralement venue d'une pièce de fabrication, d'un fil mince 3 très souple, comme du fil de suture. Ce fil mince 3 est de préférence également résorbable et généralement formé de la même matière que le lien 1.

Ce fil 3 est fixé à son extrémité libre à une aiguille 4 permettant la mise en place du lien 1 du dispositif.

Il est évident que le présent dispositif est réalisé dans plusieurs tailles car pour une mise en place aisée il est préférable que la courbure et la longueur de l'aiguille 4 correspondent à la courbure de l'anneau de l'orifice valvulaire et à la longueur de la portion de périmètre de l'orifice devant être équipé du lien 1 du dispositif.

De même, il est préférable que le lien 1 présente une longueur et si possible une courbure correspondant à la portion de la périphérie de l'orifice valvulaire devant être équipé du lien.

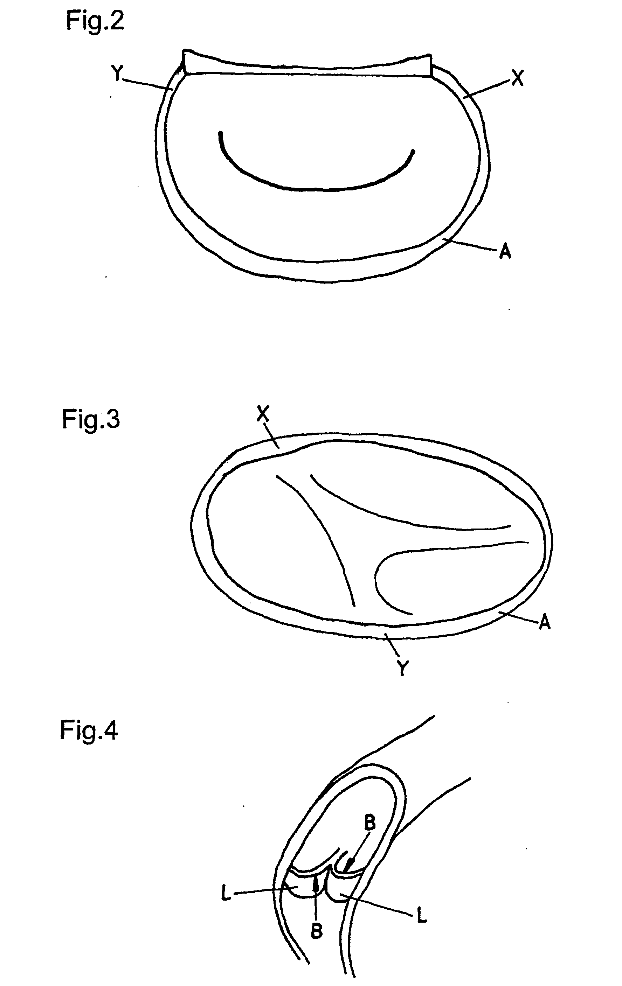

Ainsi, le chirurgien peut, dans le cas des valves illustrées aux figures 2 et 3, introduire l'aiguille 4 en X dans l'endomyocarde de l'anneau valvulaire A, faire cheminer l'aiguille à l'intérieur de cette couche de tissus jusqu'au point Y de l'anneau valvulaire A puisque la courbure de l'aiguille 4 et sa longueur sont adaptées à la valve devant être équipée.

Le chirurgien ressort l'aiguille 4 au point Y et tire le fil 3 pour entraîner le lien 1 en position pour laquelle l'organe de fixation 2 est situé à proximité du point d'introduction X et la jonction entre le lien 1 et le fil mince 3 est elle située au point de sortie Y. Le chirurgien fixe en faisant quelques points de suture et en utilisant éventuellement un arrêt, par exemple un bouton, le lien 1 au point de sortie Y avec le fil 3, puis coupe ce fil 3. Enfin, le chirurgien fixe et enfouit dans l'endomyocarde par quelques points de suture la boucle 2 ou l'arrêt prévu à l'extrémité libre du lien 1.

Il est également possible d'équiper la totalité du périmètre de l'anneau valvulaire avec le lien 1. Dans ce cas, les points X et Y sont proches ou confondus et les fils 3 munissant les deux extrémités du lien 1 sont noués ensemble, coupés et enfouis dans l'endomyocarde.

Le lien 1 présente une courbure correspondant approximativement à celle de l'anneau de l'orifice de la valve et la quantité de matière résorbable dépend de la masse de tissus fibreux que l'on veut induire par la résorption pour obtenir la rigidité désirée de cette cicatrice naturelle qui va à long terme assurer seule le maintien de l'anneau de l'orifice de la valve et empêcher toute dilatation de celui-ci.

Plusieurs tailles du dispositif sont prévues en fonction du diamètre de l'orifice valvulaire et de la surface corporelle du patient, le lien 1 et l'aiguille 4 dépendent de ce diamètre et de la portion X - Y de la périphérie de l'orifice valvulaire devant être équipée. Par ailleurs, pour chacune des tailles, plusieurs types sont prévus avec des épaisseurs différentes du lien 1.

Dans une variante du dispositif décrit, le lien 1 en matériau résorbable est revêtue d'une couche d'un second matériau plus rapidement résorbable que celui utilisé pour réaliser la partie interne du lien. De cette manière, on obtient de par la résorption de cette couche ou revêtement, une résorption initiale rapide, par exemple de quelques jours à quelques semaines, et donc la formation plus rapide d'un tissu fibreux permettant un renforcement quasi immédiat de l'orifice valvulaire. Cette cicatrisation initiale rapide est suivie d'une cicatrisation lente, six à douze mois, due à la résorption de la partie centrale du lien 1.

Pour déterminer la taille du dispositif devant être utilisé, le chirurgien dispose de testeurs, gabarits de la forme des orifices valvulaires, mais de sections différentes. En choisissant un testeur correspondant à la taille de la surface du feuillet antérieur de la valve mitrale ou tricuspide à réparer ou au diamètre de la jonction sinotubulaire où les trois valvules sigmoïdes se coaptent, le chirurgien détermine la taille du dispositif à utiliser. Le choix du type de dispositif à l'intérieur de la taille déterminée s'apprécie en fonction de l'âge du patient, de sa surface corporelle, et de l'état de la lésion. Plus la quantité de matière résorbable du lien 1 est grande, plus la cicatrice sera importante et plus fort sera le renforcement de l'anneau de l'orifice valvulaire.

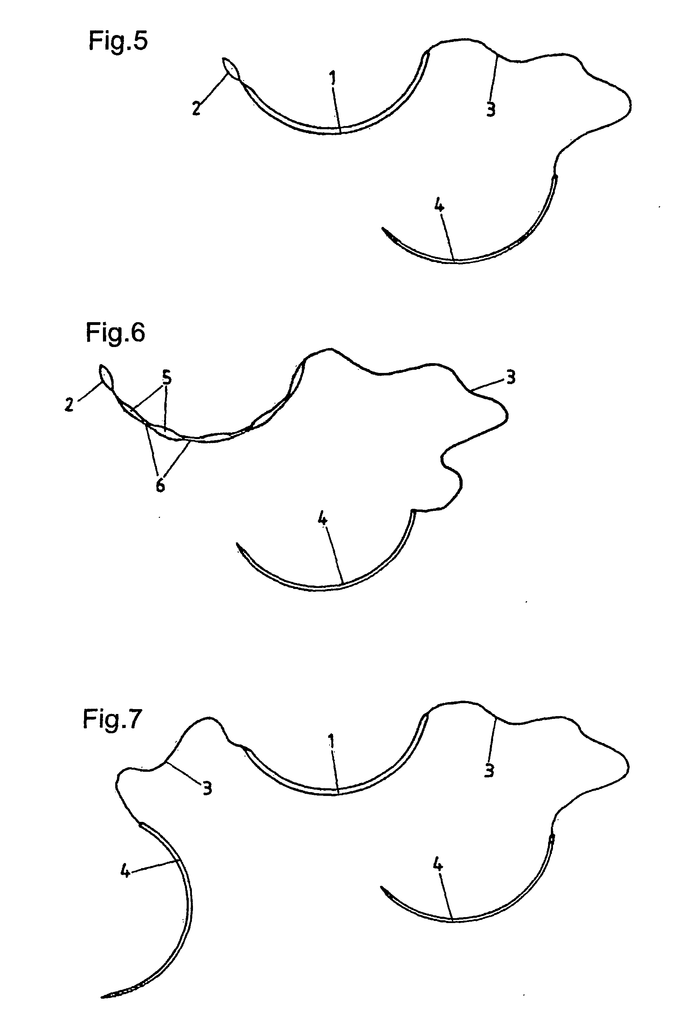

Dans la seconde forme d'exécution du dispositif illustré à la figure 6, le lien présente une succession de renflements 5 et d'amincissements 6. Ce type de dispositif engendre une cicatrisation légère ou faible au niveau des amincissements 6 et forte au niveau des renflements 5. Ceci est particulièrement intéressant chez les jeunes enfants ou les nourrissons car lors de la croissance les parties à faible cicatrisation, correspondant aux amincissements 6, peuvent facilement s'étirer en fonction de la croissance du sujet.

Ici également, il est préférable que la courbure de l'aiguille 4 et celle du lien 5, 6 résorbable correspondent sensiblement à celle de l'orifice valvulaire à équiper.

Dans cette exécution également le lien 5, 6 résorbable peut être recouvert d'une couche de matériau plus rapidement résorbable que celui utilisé pour réaliser l'intérieur du lien de manière à créer une résorption en deux temps.

La troisième forme d'exécution du dispositif illustré à la figure 7 est plus particulièrement, mais non exclusivement, destinée au cas de réparation, rétrécissement ou renforcement d'orifices valvulaires dans lesquels il faut renforcer la totalité du pourtour de l'orifice valvulaire, par exemple dans le cas de la valve mitrale, tricuspide et sigmoïde.

Ce dispositif comporte un lien 1 épais et résorbable et dont les extrémités comportent toutes deux des fils minces 3 chacun muni d'une aiguille 4.

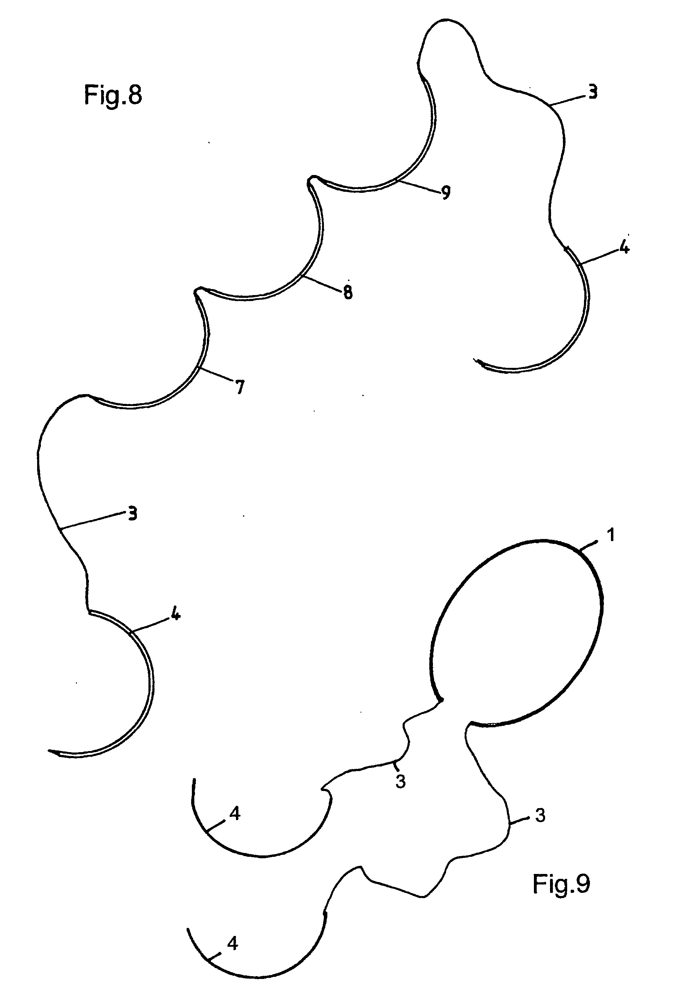

A l'aide de l'une ou l'autre aiguille 4, le chirurgien introduit le lien 1 résorbable dans l'endomyocarde de manière à former une boucle puis il noue les deux fils minces 3, également résorbables, du dispositif de manière à maintenir le lien 1 fermé sur lui-même. Le reste des fils est coupé (voir figure 9).

Ici également, la courbure des aiguilles 4 et le lien 1 du dispositif correspondent de préférence à la courbure normale de l'orifice valvulaire à équiper.

Bien entendu, le lien 1 peut comporter, comme illustré à la figure 6, des renflements et des amincissements. De même, ce lien 1 peut comporter une couche ou revêtement réalisé dans un matériau plus rapidement résorbable que celui formant l'intérieur du lien 1 du dispositif de manière à obtenir une résorption en deux temps.

La quatrième forme d'exécution du dispositif selon l'invention illustrée à la figure 8 est plus particulièrement destinée à la réparation des valvules sigmoïdes formées de trois lobes L.

Dans cette forme d'exécution, le lien est constitué de plusieurs, ici trois, parties 7, 8 ,9. La partie centrale 8 est reliée aux parties latérales 7, 9 de cette portion centrale et ces parties latérales comportent chacune un fil 3 terminé par une aiguille 4.

La courbure des aiguilles 4 et des parties 7, 8 ,9 du lien correspond à la courbure des bords libres B des lobes L de la valvule sigmoïde. A l'aide des aiguilles 4, on introduit dans l'endomyocarde le long des bords B des lobes L de la valvule les parties 7, 8, 9 de manière à ce que chacune d'elles corresponde à un lobe L.

Les fils 3 sont ensuite noués ensemble et coupés.

Ici également, le lien et éventuellement les fils 3 sont en un matériau résorbable, éventuellement en deux temps comme décrit plus haut.

Les principaux avantages du dispositif décrit sont les suivants :
- absence de prédisposition à l'infection puisque le lien implanté est biologiquement résorbable.
- facilité de mise en place du lien du fait de sa forme et de la forme des aiguilles adaptées à la courbure de l'orifice valvulaire. En fait, ceci permet d'entrer dans l'endomyocarde à un endroit et d'en ressortir à un autre ou au même endroit, sans perforation intermédiaire.
- la possibilité de créer une résorption en deux temps.
- la possibilité de créer des réparations renforçant l'anneau de l'orifice valvulaire et évitant une dilatation tout en permettant à cet anneau valvulaire de croître en fonction de la croissance du sujet, ce qui évite les sténoses tardives.

De nombreuses variantes peuvent être envisagées notamment dans la forme et la composition du dispositif et plus particulièrement de sa portion épaisse au lien.

Ce lien peut présenter un diamètre de l'ordre de 0,2 mm à quelques millimètres suivant les conditions d'utilisation. Sa section peut être circulaire, ovale, polygonale et notamment rectangulaire pour lui donner une plus grande résistance à la déformation. Ce lien est généralement souple, mais reprend de par sa propre élasticité sa forme courbe correspondant approximativement à l'orifice valvulaire.

L'un des caractères novateurs de l'invention consiste à utiliser un ou plusieurs matériaux résorbables pour les réalisations du lien 1 et des fils 3. En effet, si les matériaux résorbables connaissent plusieurs applications dans le domaine des dispositifs médicaux, par exemple en tant que fil de suture, ou en tant que prothèse, ou encore en tant que dispositif pour la libération contrôlée de substances médicamenteuses dans l'organisme, il n'existe pas d'application dans laquelle le matériau ait à assurer, outre sa fonction première d'élément réparateur, une fonction d'induction d'une action curative et évolutive provenant de l'organisme lui-même.

Les matériaux résorbables trouvant des applications dans les domaines de la santé sont obtenus à partir de tissus ou de protéines provenant du règne animal, tels que le collagène ou le catgut, ou à partir de polymères produits par voie de synthèse.

Les natures chimiques des principaux polymères connus pour être résorbables regroupent les polyesters, les polyorthoesters, les polyanhydrides, les poly (éther) esters, les polyaminoacides et les polydepsipeptides (voir par exemple : B. Buchholz ; J. Mater. Sci. Mater: Med. 4 (1993) 381 - 388).

D'une façon plus schématique, mais non exhaustive, les polymères résorbables peuvent être décrits par un motif répondant à la formule générale :

-[-X1-C(o)-R1-Y1-R2-]-[-X2-C(O)-R3-Y2-R4-]-

dans laquelle:
- C(O) désigne un groupement >C=O,
- X1 ; X2 désignent un atome d'oxygène ou un groupement NH,
- Y1 (respectivement Y2) désigne un atome d'oxygène, ou un groupement NH, ou une liaison chimique reliant directement R1 à R3 (respectivement R2 à R4),
- R1 ; R2 ; R3 ; R4 désignent des chaînes carbonées linéaires ou ramifiées, saturées ou partiellement insaturées, portant ou non des hétéroatomes et contenant de 0 à 10 atomes de carbone.

Quand dans cette formule générale, X1 est égal à X2 et Y1 est égal à Y2 et R1 est égal à R3 et R2 est égal à R4, le polymère obtenu est appelé hornopolymère. Dans le cas contraire, le polymère obtenu est appelé copolymère.

Parmi ces polymères, les inventeurs ont porté une attention toute particulière pour les polymères pouvant être décrits par un motif répondant à la formule générale: -[-X1-C(O)-R1-Y1-R2-]-[-X2-C(O)-R3-Y2-R4-]-

Dans laquelle:
- C(O) désigne un groupement >C=O,
- X1 ; X2 désignent un atome d'oxygène,
- Y1 (respectivement Y2) désigne un atome d'oxygène ou une liaison chimique reliant directement R1 à R3 (respectivement R2 à R4),
- R1 ; R2 ; R3 ; R4 désignent des chaînes carbonées linéaires ou ramifiées et contenant de 0 à 5 atomes de carbone et de préférence de 0 à 3 atomes.

Ces types de polymères incluent par exemple les polylactides, les polyglycolides, les polydioxanones, les polyalkylènecarbonates et les polylactones. A ces homopolymères s'ajoutent encore les copolymères obtenus par combinaisons des différents monomères.

Ces polymères sont connus pour leur aptitude à se résorber in vivo selon des modes de résorption connus et prévisibles.

En outre, parmi ces polymères, certains vont présenter des caractéristiques particulièrement intéressantes pour entrer dans la fabrication du dispositif tel que décrit dans la revendication 1.

Ainsi, par exemple, les polydioxanones sont connus pour se résorber plus lentement que les polylactides, ou les polyglycolides, ou encore que le catgut ou le collagène.

D'un autre côté, la souplesse du matériau obtenu dépend également de la nature du polymère utilisé. Les caractéristiques mécaniques du matériau obtenu varieront par exemple avec la nature chimique du motif, le poids moléculaire, le procédé de polymérisation, la technique de mise en oeuvre du matériau, ...

L'optimisation des différents paramètres influant sur les caractéristiques du matériau obtenu a conduit à préférer les polydioxanones pour réaliser le lien 1. Les polydioxanones sont les polymères obtenus à partir de monomères cycliques répondant à la formule brute C₄H₆O₃ et possédant un groupement >C=O. Ils offrent une cinétique de résorption in vivo compatible avec la formation du bourrelet cicatriciel et peuvent être développés avec les caractéristiques mécaniques nécessaires à leur mise en oeuvre.

Dans le cas où le lien 1 est formé de deux matériaux différents, on peut prévoir que celui-ci comporte une partie interne en polydioxanone et un revêtement externe réalisé dans un polymère résorbable à cinétique plus rapide. Le revêtement externe provoque une première réaction fibreuse pendant sa résorption précoce tout en protégeant le polydioxanone qui ne débutera sa résorption lente que lorsque le revêtement externe sera résorbé. On obtient ainsi une résorption plus tardive qui induit une réaction fibreuse plus consolidante.

Dans le cas où le lien comporte des renflements et des amincissements, les segments principaux épais 5 comporteraient les deux matériaux tandis que les parties minces de liaison 6 pourraient ne comporter que l'un des deux matériaux précités.

Plutôt que d'utiliser des matériaux monofilament pour le lien, on peut utiliser des matériaux tressés ou multifibres.

## Revendications

1. Dispositif pour le rétrécissement et/ou le renforcement des orifices valvulaires du coeur comportant un lien (1) épais en un matériau résorbable, (bioabsorbable ou biodégradable), élastique et courbe, solidaire à l'une de ses extrémités au moins d'un fil (3) mince dont l'extrémité est fixée à une aiguille courbe (4), **caractérisé par le fait que** le fil (3) est agencé de manière à ce qu'il permette d'exercer une traction longitudinale sur le lien (1) épais dans la direction de l'axe de ce lien (1) épais, la traction longitudinale étant adaptée pour permettre que le lien (1) épais peut être introduit à l'intérieur du tissu de l'orifice valvulaire à équiper.

2. Dispositif selon la revendication 1, **caractérisé par le fait que** la courbure de l'aiguille (4) est sensiblement égale à celle du lien (1) et que cette courbure correspond sensiblement à celle de l'orifice valvulaire à équiper, cette courbure étant adaptée pour permettre que le lien (1) épais peut être introduit à l'intérieur du tissu de l'orifice valvulaire à équiper.

3. Dispositif selon la revendication 1 ou 2, **caractérisé par le fait que** l'autre extrémité du lien (1) est également solidaire d'un fil (3) dont l'extrémité est fixée à une seconde aiguille courbe (4).

4. Dispositif selon l'une des revendications 1 à 3, **caractérisé par le fait que** le ou les fils (3) sont également en un matériau résorbable.

5. Dispositif selon l'une des revendications1 à 4, **caractérisé par le fait que** la longueur de l'aiguille (4) est sensiblement égale à la longueur du lien (1), correspondant sensiblement à la portion du pourtour de l'orifice valvulaire dans laquelle il doit être implanté.

6. Dispositif selon l'une des revendications 1 à 2 et 4 à 5, **caractérisé par le fait que** l'extrémité libre du lien (1) comporte un organe de fixation (2).

7. Dispositif selon l'une des revendications précédentes, **caractérisé par le fait que** la section du lien (1) est circulaire ou polygonale.

8. Dispositif selon l'une des revendications précédentes, **caractérisé par le fait que** le lien (1) comporte une âme réalisée dans un polymère pouvant être décrit par le motif général -[-X-C(O)-R1-Y-R2-]- dans lequel C(O) désigne un groupement >C=O ; X désigne un atome d'oxygène, Y désigne un atome d'oxygène ou une liaison chimique reliant R1 à R2 ; R1 et R2 désignent des chaînes carbonées linéaires ou ramifiées et contenant de 0 à 5 atomes de carbone et de préférence de 0 à 3 atomes.

9. Dispositif selon la revendication 8, **caractérisé par le fait que** le polymère utilisé est de type polylactide, ou polyglycolide, ou polylactone, ou polyalkylènecarbonate, ou encore, et de préférence, de type polydioxanone ou de tout autre type de polymère pourvu que ce dernier soit obtenu à partir d'un composé cyclique répondant à la formule brute C₄H₆O₃ et possédant un groupement >C=O.

10. Dispositif selon l'une des revendications précédentes, **caractérisé par le fait que** le lien (1) comporte une âme en un matériau à résorption lente revêtue d'une couche d'un matériau à résorption plus rapide.

11. Dispositif selon la revendication 10, **caractérisé par le fait que** le polymère utilisé pour constituer l'âme du lien (1) est un copolymère obtenu par combinaison des différents monomères conduisant aux polymères revendiqués dans les revendications 8 et 9.

12. Dispositif selon la revendication 10 ou 11, **caractérisé par le fait que** le matériau à résorption lente est un des polymères revendiqués dans les revendications 8 à 10 et que le matériau à résorption plus rapide est un des polymères revendiqués dans les revendications 8 à 10 auxquels s'ajoutent le collagène et le catgut.

13. Dispositif selon la revendication 12, **caractérisé par le fait que** le matériau à résorption lente est un polymère de type polydioxanone ou de tout autre type pourvu que le polymère soit obtenu à partir d'un composé cyclique répondant à la formule brute C₄H₆O₃ et possédant un groupement >C=O et que le matériau à résorption plus rapide est un des polymères revendiqués dans les revendications 8 à 10 auxquels s'ajoutent le collagène et le catgut.

14. Dispositif selon l'une des revendications précédentes, **caractérisé par le fait que** le lien comporte une succession de renflements (5) et d'amincissements (6).

15. Dispositif selon les revendications 10 à 14, **caractérisé par le fait que** les renflements (5) du lien comportent une première matière résorbable recouverte d'une seconde matière résorbable tandis que les zones amincies (6) du lien sont constituées d'une seule matière résorbable.

## Patentansprüche

1. Vorrichtung zur Verengung und/oder Verstärkung der Herzklappenöffnungen mit einem dicken Band (1) aus einem resorbierbaren (bioabsorbierbaren oder biodegradierbaren), elastischen und gekrümmten Material, das zumindest an einem seiner Enden fest mit einem dünnen Faden (3) verbunden ist, dessen Ende an einer gekrümmten Nadel (4) befestigt ist, **dadurch gekennzeichnet, dass** der Faden (3) so beschaffen ist, dass er einen Längszug auf das dicke Band (1) in der Richtung der Achse dieses dicken Bandes (1) auszuüben gestattet, wobei der Längszug es zu ermöglichen vermag, dass das dicke Band (1) ins Gewebeinnere der auszurüstenden Herzklappenöffnung eingeführt werden kann.

2. Vorrichtung nach Anspruch 1, **dadurch gekennzeichnet, dass** die Krümmung der Nadel (4) im Wesentlichen gleich der des Bandes (1) ist, und **dadurch**, dass diese Krümmung im Wesentlichen der der auszurüstenden Herzklappenöffnung entspricht, wobei diese Krümmung es zu ermöglichen vermag, dass das dicke Band (1) ins Gewebeinnere der auszurüstenden Herzklappenöffnung eingeführt werden kann.

3. Vorrichtung nach Anspruch 1 oder 2, **dadurch gekennzeichnet, dass** das andere Ende des Bandes (1) ebenfalls fest mit einem Faden (3) verbunden ist, dessen Ende an einer zweiten gekrümmten Nadel (4) befestigt ist.

4. Vorrichtung nach einem der Ansprüche 1 bis 3, **dadurch gekennzeichnet, dass** der oder die Fäden (3) ebenfalls aus einem resorbierbaren Material bestehen.

5. Vorrichtung nach einem der Ansprüche 1 bis 4, **dadurch gekennzeichnet, dass** die Länge der Nadel (4) im Wesentlichen gleich der Länge des Bandes (1) ist und im Wesentlichen dem Abschnitt der Peripherie der Herzklappenöffnung entspricht, in den es implantiert werden soll.

6. Vorrichtung nach einem der Ansprüche 1 bis 2 und 4 bis 5, **dadurch gekennzeichnet, dass** das freie Ende des Bandes (1) ein Befestigungselement (2) trägt.

7. Vorrichtung nach einem der vorangehenden Ansprüche, **dadurch gekennzeichnet, dass** der Querschnitt des Bandes (1) kreisförmig oder polygonal ist.

8. Vorrichtung nach einem der vorangehenden Ansprüche, **dadurch gekennzeichnet, dass** das Band (1) eine Seele aufweist, die aus einem Polymer hergestellt ist, das durch die allgemeine Gruppierung ―[―X―C(O)―R1―Y―R2―]― beschrieben werden kann, in der C(O) eine Gruppe >C=O bezeichnet, X ein Sauerstoffatom bezeichnet, Y ein Sauerstoffatom oder eine chemische Bindung bezeichnet, die R1 mit R2 verbinden; R1 und R2 gerade oder verzweigte Kohlenstoffketten bezeichnen, die 0 bis 5 Kohlenstoffatome und bevorzugt 0 bis 3 Kohlenstoffatome enthalten.

9. Vorrichtung nach Anspruch 8, **dadurch gekennzeichnet, dass** das eingesetzte Polymer vom Typ eines Polylactids, Polyglycolids, Polylactons oder Polyalkylencarbonats oder bevorzugt noch vom Typ eines Polydioxanons oder irgendeinem anderen Polymertyp ist, solange dieses Polymer ausgehend von einer cyclischen Verbindung erhalten wird, die der Bruttoformel C₄H₆O₃ entspricht und eine >C=O-Gruppe besitzt.

10. Vorrichtung nach einem der vorangehenden Ansprüche, **dadurch gekennzeichnet, dass** das Band (1) eine Seele aus einem langsam resorbierbaren Material besitzt, das mit einer Schicht aus schneller resorbierbarem Material überzogen ist.

11. Vorrichtung nach Anspruch 10, **dadurch gekennzeichnet, dass** das zur Herstellung der Seele des Bandes (1) verwendete Polymer ein Copolymer ist, das durch Kombination der verschiedenen Monomere gewonnen wird, die zu den Polymeren führen, die in Ansprüchen 8 und 9 beansprucht werden.

12. Vorrichtung nach Anspruch 10 oder 11, **dadurch gekennzeichnet, dass** das langsam resorbierbare Material eines der in den Ansprüchen 8 bis 10 beanspruchten Polymere ist und dass das schneller resorbierbare Material eines der in den Ansprüchen 8 bis 10 beanspruchten Polymere ist, zu denen Kollagen und Katgut hinzukommen.

13. Vorrichtung nach Anspruch 12, **dadurch gekennzeichnet, dass** das langsam resorbierbare Material ein Polymer vom Typ des Polydioxanons oder irgendeines anderen Typs ist, solange das Polymer ausgehend von einer cyclischen Verbindung gewonnen wird, die der Bruttoformel C₄H₆O₃ entspricht und eine >C=O-Gruppe besitzt, während das schneller resorbierbare Material eines der in den Ansprüchen 8 bis 10 beanspruchten Polymere ist, zu denen Kollagen und Katgut hinzukommen.

14. Vorrichtung nach einem der vorangehenden Ansprüche, **dadurch gekennzeichnet, dass** das Band eine Folge von Verdickungen (5) und Verdünnungen (6) aufweist.

15. Vorrichtung nach Ansprüchen 10 bis 14, **dadurch gekennzeichnet, dass** die Verdickungen (5) des Bandes ein erstes resorbierbares Material aufweisen, das mit einem zweiten resorbierbaren Material überzogen ist, während die verdünnten Bereiche (6) des Bandes aus nur einem resorbierbaren Material bestehen.

## Claims

1. Device for contracting and/or reinforcing valvular orifices of the heart, comprising a thick connection (1) of a resorbable material (bioabsorbable or biodegradable), flexible and curved, secured at one of its ends to at least one thin filament (3) whose end is fixed to a curved needle (4), **characterized by** the fact that the filament (3) is arranged such that it permits exerting a longitudinal traction on the thick connection (1) in the direction of the axis of this thick connection (1), this longitudinal traction being adapted to allow the thick connection (1) to be introduced inside the tissue of the valvular orifice to be treated.

2. Device according to claim 1, **characterized by** the fact that the curvature of the needle (4) is substantially equal to that of the connection (1) and that this curvature corresponds substantially to that of the valvular orifice to be treated, this curvature being adapted to allow the thick connection (1) to be introduced inside the tissue of the valvular orifice to be treated.

3. Device according to claim 1 or 2, **characterized by** the fact that the other end of the connection (1) is also secured to a filament (3) whose end is fixed to a second curved needle (4).

4. Device according to one of claims 1 to 3, **characterized by** the fact that the filament or filaments (3) are also of a resorbable material.

5. Device according to one of claims 1 to 4, **characterized by** the fact that the length of the needle (4) is substantially equal to the length of the connection (1), corresponding substantially to the portion of the periphery of the valvular orifice into which it is to be implanted.

6. Device according to one of claims 1 to 2 and 4 to 5, **characterized by** the fact that the free end of the connection (1) comprises a securement member (2).

7. Device according to one of the preceding claims, **characterized by** the fact that the cross-section of the connection (1) is circular or polygonal.

8. Device according to one of the preceding claims, **characterized by** the fact that the connection (1) comprises a core made of a polymer that can be described by the general formula -[-X-C(O)-R1-Y-R2-]- in which C(O) designates a >C=O group; X designates an oxygen atom, Y designates an oxygen atom or a chemical bond connecting R1 to R2; R1 and R2 designating linear or branched carbon chains and containing 0 to 5 carbon atoms and preferably 0 to 3 carbon atoms.

9. Device according to claim 8, **characterized by** the fact that the polymer used is of the polylactide or polyglycolide or polylactone or polyalkylenecarbonate type or else preferably of the polydioxanone type or any other type of polymer, provided that this latter is obtained from a cyclic compound having the general formula C₄H₆O₃ and having a >C=O group.

10. Device according to one of the preceding claims, **characterized by** the fact that the connection (1) comprises a core of a slowly resorbable material covered by a layer of a more rapidly resorbable material.

11. Device according to claim 10, **characterized by** the fact that the polymer used to constitute the core of the connection (1) is a copolymer obtained by combination of different monomers leading to the polymers claimed in claims 8 and 9.

12. Device according to claim 10 or 11, **characterized by** the fact that the slowly resorbable material is one of the polymers claimed in claims 8 to 10 and that the more rapidly resorbable material is one of the polymers claimed in claims 8 to 10 to which are added collagen and catgut.

13. Device according to claim 12, **characterized by** the fact that the slowly resorbable material is a polymer of the polydioxanone type or of any other type provided that the polymer is obtained from a cyclic compound responding to the general formula C₄H₆O₃ and having a >C=O group and that the more rapidly resorbable material is one of the polymers claimed in claims 8 to 10 to which are added collagen and catgut.

14. Device according to one of the preceding claims, **characterized by** the fact that the connection comprises a series of enlargements (5) and thinned portions (6).

15. Device according to claims 10 to 14, **characterized by** the fact that the enlargements (5) of the connection comprise a first resorbable material covered with a second resorbable material whilst the thinned regions (6) of the connection are constituted by a single resorbable material.
